# EUROPÄISCHE PATENTSCHRIFT

(11) **EP 3 378 537 B1**
(45) Veröffentlichungstag und Bekanntmachung des Hinweises auf die Patenterteilung: **28.08.2019**
(21) Anmeldenummer: 18160121.2
(22) Anmeldetag: 06.03.2018
(51) Int. Cl.: A61Q 17/04, A61K 8/81

(54) **SONNENSCHUTZMITTEL MIT TRIACONTANYL PVP**
SUNSCREEN AGENT COMPRISING TRIACONTANYL PVP
AGENT PHOTOPROTECTEUR CONTENANT DU TRIACONTANYL PVP

(30) Priorität: 22.03.2017 DE 102017204794
(43) Veröffentlichungstag der Anmeldung: 26.09.2018
(73) Patentinhaber: Beiersdorf AG, 20253 Hamburg (DE)
(72) Erfinder: Eisert, Anja, 22085 Hamburg (DE); Sprock, Sarah, 22297 Hamburg (DE); Göddertz, Dominik, 22549 Hamburg (DE)

(56) Entgegenhaltungen:
- EP-A1- 2 939 710
- Anonymous: "Everything on SPF and UV protection in Natural Oils, Herbs and Foods you need to know", Worldnaturelle, 14. März 2017 (2017-03-14), XP55493344, Gefunden im Internet: URL:https://worldnaturelle.wordpress.com/2 015/08/07/everything-on-spf-and-uv-protect ion-in-natural-oils-herbs-and-foods-you-ne ed-to-know/ [gefunden am 2018-07-18]
- ANONYMOUS: "Symmetrical Triazine derivatives", IP.COM JOURNAL, IP.COM INC., WEST HENRIETTA, NY, US, 20. September 2004 (2004-09-20), XP013021505, ISSN: 1533-0001

## Beschreibung

Die vorliegende Erfindung betrifft eine kosmetische Zubereitung enthaltend ein oder mehrere UV-Filter, Polyglyceryl-10 Stearat (INCI Polyglyceryl-10 Stearate) und 1-Ethenyl-pyrrolidin-2-on Polymer mit 1-Triaconten (INCI Triacontanyl PVP, CAS 136445-69-7), sowie ein Verfahren zur Erhöhung der Auswaschbarkeit von UV-Filtern aus Textilien, die mit einer kosmetischen Zubereitung, welche diese UV-A Filter und/oder Breitbandfilter enthält, kontaminiert sind, wobei der Zubereitung Triacontanyl PVP zugesetzt wird und die Verwendung von Triacontanyl PVP in UV-Lichtschutzfilter enthaltenden kosmetischen Zubereitungen zur Erleichterung der Auswaschbarkeit der UV-Lichtschutzfilter aus mit den Zubereitungen kontaminierten Textilien.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Kosmetische Zubereitungen wie Sonnenschutzzubereitungen, die auf die Haut aufgetragen werden, kommen regelmäßig (beabsichtigt oder unbeabsichtigt) mit Kleidungsstücken und Wäschestücken (z.B. Handtücher) in Kontakt, an denen sie (z.B. als "Abrieb" oder weil sie von den Faserstoffen "aufgesaugt" werden) zum Teil haften bleiben. Auf diese Weise entstehen, je nach Art der Inhaltsstoffe, insbesondere auf hellen Textilien Flecken und Verfärbungen. Diese Verfärbungen werden insbesondere durch nicht-wasserlösliche UVA- und Breitbandfilter wie 4-(tert.-Butyl)-4'-methoxydibenzoylmethan (INCI Butyl Methoxydibenzoylmethane), (2-[-4-(Diethylamino)-2-hydroxybenzoyl]Benzoesäurehexylester (INCI: Diethylamino Hydroxybenzoyl Hexyl Benzoate) und 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) hervorgerufen. Die Verfleckungen sind durch Waschen mit herkömmlichen Waschmitteln kaum zu entfernen und verstärken sich während des Waschprozesses durch Wechselwirkungen mit Ionen des Waschwassers sogar noch.

Es war daher die Aufgabe der vorliegenden Erfindung, die Nachteile des Standes der Technik zu beseitigen und eine kosmetische Zubereitung (insbesondere ein Sonnenschutzmittel) zu entwickeln, die nicht-wasserlösliche UV-A-Filter wie 4-(tert.-Butyl)-4'-methoxydibenzoylmethan (INCI Butyl Methoxydibenzoylmethane) und (2-[-4-(Diethylamino)-2-hydroxybenzoyl] Benzoesäurehexylester (INCI: Diethylamino Hydroxybenzoyl Hexyl Benzoate) sowie gegebenenfalls Breitbandfilter wie Bis-Ethylhexyloxyphenol Methoxyphenyl Triazine enthält, welche sich leichter aus den mit der Zubereitung kontaminierten Textilen herauswaschen lassen.

Überraschend gelöst werden die Aufgaben durch ein Verfahren zur Erhöhung der Auswaschbarkeit von Hexyl 2-[4-(diethylamino)-2-hydroxybenzoyl]benzoate (INCI: Diethylamino hydroxybenzoyl hexyl benzoate), 4-(tert.-Butyl)-4'-methoxydibenzoylmethan und/oder 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) aus Textilien, die mit einer kosmetischen Zubereitung, welche diese UV-A Filter und/oder Breitbandfilter enthält, kontaminiert sind, dadurch gekennzeichnet, dass der kosmetischen Zubereitung 1-Ethenyl-pyrrolidin-2-on Polymer mit 1-Triaconten (INCI Triacontanyl PVP, CAS 136445-69-7) zugesetzt wird.

Überraschend gelöst werden die Aufgaben ferner durch ein Verfahren zur Reduzierung der durch UV-Lichtschutzfilter Hexyl 2-[4-(diethylamino)-2-hydroxybenzoyl]benzoate (INCI: Diethylamino hydroxybenzoyl hexyl benzoate), 4-(tert.-Butyl)-4'-methoxydibenzoylmethan und/oder 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) enthaltenden kosmetischen Zubereitungen hervorgerufenen Textilverfleckung, dadurch gekennzeichnet, dass dem Kosmetikum Zubereitung 1-Ethenyl-pyrrolidin-2-on Polymer mit 1-Triaconten (INCI Triacontanyl PVP, CAS 136445-69-7) zugesetzt wird.
Überraschend gelöst werden die Aufgaben darüber hinaus durch ein Verwendung einer Mischung aus 1-Ethenyl-pyrrolidin-2-on Polymer mit 1-Triaconten (INCI Triacontanyl PVP, CAS 136445-69-7) in die UV-Lichtschutzfilter Hexyl 2-[4-(diethylamino)-2-hydroxybenzoyl]benzoate (INCI: Diethylamino hydroxybenzoyl hexyl benzoate), 4-(tert.-Butyl)-4'-methoxydibenzoylmethan und/oder 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) enthaltenden kosmetischen Zubereitungen zur Erleichterung der Auswaschbarkeit der UV-Lichtschutzfilter aus mit den Zubereitungen kontaminierten Textilien.

Überraschend gelöst werden die Aufgaben nicht zuletzt durch eine kosmetische Zubereitung enthaltend
a) ein oder mehrere UV-Filter, gewählt aus der Gruppe der Verbindungen Hexyl 2-[4-(diethylamino)-2-hydroxybenzoyl]benzoate (INCI: Diethylamino hydroxybenzoyl hexyl benzoate), 4-(tert.-Butyl)-4'-methoxydibenzoylmethan, 2,4-Bis-{[4-(2-ethyl-hexyloxy)-2-hydro-xy]-phenyl}-6-(4-methoxyphenyl)-1,3,5-triazin (INCI: Bis- Ethylhexyloxyphenol methoxyphenyl Triazine),
b) Polyglyceryl-10 Stearat (INCI Polyglyceryl-10 Stearate),
c) 1-Ethenyl-pyrrolidin-2-on Polymer mit 1-Triaconten (INCI Triacontanyl PVP, CAS 136445-69-7).

Zwar kennt der Stand der Technik die DE 102014216602, DE 102014202956, DE 102013213170, DE 102013200819, DE 102011088962 und EP 2937073. Auch gibt es den nachveröffentlichten Stand der Technik der DE102017200723 doch konnten diese Schriften nicht den Weg zur vorliegenden Erfindung weisen. Nicht zuletzt kennt der Fachmann den Internet-Eintrag "everything on SPF and UV protection in Natural Oils, Herbs and Food you need to know": https://worldnaturelle.wordpress.com/2015/08/07/everything-on-spf-and-uvprotection-in-natural-oils-herbs-and-foods-you-need-to-know/, die EP2939710 und.die Veröffentlichung "Symetrical Triazine derivatives", IP.COM Journal vom 20.September 2004, XP013021505, ISSN: 1533-0001, die ebenfalls nicht den Weg zur vorliegenden Erfindung weisen konnten.

Im Rahmen der vorliegenden Offenbarung beziehen sich die Formulierungen "erfindungsgemäß", "erfindungsgemäße Zubereitung" etc. immer auf die erfindungsgemäßen Zubereitungen, Verfahren und Verwendungen, d.h. auch auf Zubereitungen, in denen die erfindungsgemäßen Verwendungen verwirklicht werden sowie Zubereitungen, mit denen das erfindungsgemäße Verfahren verwirklicht wird.

Es ist erfindungsgemäß vorteilhaft, wenn die erfindungsgemäße Zubereitung in Form einer Emulsion vorliegt. Dabei ist es erfindungsgemäß bevorzugt, wenn die Zubereitung in Form einer Öl-in-Wasser-Emulsion (O/W-Emulsion) vorliegt. Erfindungsgemäß besonders bevorzugt liegt die O/W-Emulsion in Form einer Lotion vor.

Es ist erfindungsgemäß vorteilhaft, wenn die erfindungsgemäße Zubereitung 1-Ethenyl-pyrrolidin-2-on Polymer mit 1-Triaconten (INCI Triacontanyl PVP, CAS 136445-69-7) in einer Konzentration von 0,01 bis 3 Gewichts-%, bezogen auf das Gesamtgewicht der Zubereitung enthält. Die erfindungsgemäß bevorzugte Einsatzkonzentration liegt dabei zwischen 0,1 und 2,5 Gewichts-%, bezogen auf das Gesamtgewicht der Zubereitung.

Vorteilhafte Ausführungsformen der vorliegenden Erfindung sind dadurch gekennzeichnet, dass die Zubereitung Polyglyceryl-10 Stearat in einer Konzentration von 0,01 bis 2 Gewichts-%, bezogen auf das Gesamtgewicht der Zubereitung enthält. Die erfindungsgemäß bevorzugte
Einsatzkonzentration liegt dabei zwischen 0,1 und 1 Gewichts-%, bezogen auf das Gesamtgewicht der Zubereitung

Vorteilhaft im Sinne der vorliegenden Erfindung sind Zubereitungen, die dadurch gekennzeichnet sind, dass die Zubereitung Phenoxyethanol enthält.
In einem solchen Fall ist es erfindungsgemäß von Vorteil Phenoxyethanol in einer Konzentration von 0,1 bis 1 Gewichts-%, bezogen auf das Gesamtgewicht der Zubereitung, einzusetzen.

Erfindungsgemäß vorteilhaft ist es, wenn die Zubereitung Cellulosegummi (INCI Cellulose gum) und/oder Xanthangummi (INCI Xanthan gum) enthält. Dabei ist ein Gehalt von Cellulosegummi und Xanthangummi erfindungsgemäß bevorzugt.

Es ist es erfindungsgemäß von Vorteil Cellulosegummi (INCI Cellulose gum) in einer Konzentration von 0,01 bis 1,5 Gewichts-%, bezogen auf das Gesamtgewicht der Zubereitung, einzusetzen.

Die erfindungsgemäß vorteilhafte Einsatzkonzentration für Xanthangummi beträgt von 0,1 bis 0,6 Gewichts-%, bezogen auf das Gesamtgewicht der Zubereitung.

Erfindungsgemäß vorteilhafte Ausführungsformen der vorliegenden Erfindung sind dadurch gekennzeichnet, dass die Zubereitung Tetranatriumiminodisuccinat (INCI Tetrasodium Iminodisuccinate) enthält.

Es ist erfindungsgemäß vorteilhaft, wenn die erfindungsgemäße Zubereitung Tetranatriumiminodisuccinat (INCI Tetrasodium Iminodisuccinate) in einer Konzentration von 0,01 bis 1 Gewichts-%, bezogen auf das Gesamtgewicht der Zubereitung enthält. Die erfindungsgemäß bevorzugte Einsatzkonzentration für Tetranatriumiminodisuccinat (INCI Tetrasodium Iminodisuccinate) liegt bei einer Konzentration von 0,05 bis 0,8 Gewichts-%, bezogen auf das Gesamtgewicht der Zubereitung.

Erfindungsgemäß ganz besonders bevorzugt ist es, eine Kombination aus Cellulosegummi (INCI Cellulose gum) und Tetranatriumiminodisuccinat (INCI Tetrasodium Iminodisuccinate) einzusetzen, wobei die oben genannten Konzentrationsbereiche die erfindungsgemäß bevorzugten Einsatzkonzentrationen darstellen.

Vorteilhaft im Sinne der vorliegenden Erfindung sind Zubereitungen, die dadurch gekennzeichnet sind, dass die Zubereitung Ethylhexylglycerin, Propylenglycol, Butylenglycol, 2-Methylpropan-1,3-diol, 1,2-Pentandiol, 1,2-Hexandiol, 1,2-Octandiol und/oder 1,2-Decandiol enthält.

Dabei ist insbesondere der Einsatz von Ethylhexylglycerin erfindungsgemäß besonders vorteilhaft.

Die erfindungsgemäß vorteilhafte Einsatzkonzentration für Ethylhexylglycerin beträgt von 0,1 bis 0,6 Gewichts-%, bezogen auf das Gesamtgewicht der Zubereitung.

Erfindungsgemäß bevorzugt ist der Einsatz einer Kombination aus Phenoxyethanol und Ethylhexylglycerin.

Es ist vorteilhaft im Sinne der vorliegenden Erfindung, wenn die erfindungsgemäße Zubereitung Ethanol und/oder Glycerin enthält. Ethanol wird dabei erfindungsgemäß vorteilhaft in einer Konzentration von 1 bis 10 Gewichts-%, bezogen auf das Gesamtgewicht der Zubereitung eingesetzt, Glycerin in einer Konzentration von 0,5 bis 10 Gewichts-%, bezogen auf das Gesamtgewicht der Zubereitung.

Es ist erfindungsgemäß vorteilhaft, wenn die Zubereitung einen oder mehrere UV-Filter gewählt aus der Gruppe der Verbindungen Hexyl 2-[4-(diethylamino)-2-hydroxybenzoyl]benzoate (INCI: Diethylamino hydroxybenzoyl hexyl benzoate), 4-(tert.-Butyl)-4'-methoxydibenzoylmethan, 2-Phenylbenzimidazol-5-sulfonsäuresalzen, 2,4,6-Tris-[anilino-(p-carbo-2'-ethyl-1'-hexyloxy)]-1,3,5-triazin (INCI: Ethylhexyl Triazone), 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine), 4,4'-[[6-[[4-[[(1,1 dimethylethyl)amino]carbonyl]phenyl]amino]-1,3,5-triazine-2,4-diyl]diimino]bis-, bis(2-ethylhexyl)benzoat (INCI: Diethylhexyl Butamido Triazone), 2-Ethylhexyl 2-hydroxybenzoat (INCI: Ethylhexyl Salicylate), 3,3,5-Trimethylcyclohexyl 2-hydroxybenzoat (INCI: Homosalate), Titandioxid enthält.

Erfindungsgemäß bevorzugt ist es, wenn die Zubereitung einen oder mehrere UV-Filter gewählt aus der Gruppe der Verbindungen Hexyl 2-[4-(diethylamino)-2-hydroxybenzoyl]benzoate (INCI: Diethylamino hydroxybenzoyl hexyl benzoate), 4-(tert.-Butyl)-4'-methoxydibenzoylmethan, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine), enthält.

Enthält die erfindungsgemäße Zubereitung Hexyl 2-[4-(diethylamino)-2-hydroxybenzoyl]benzoate (INCI: Diethylamino hydroxybenzoyl hexyl benzoate), so ist es erfindungsgemäß vorteilhaft, diese Verbindung in einer Konzentration von 0,25 bis 5 Gewichts-%, bezogen auf das Gesamtgewicht der Zubereitung einzusetzen.

Enthält die erfindungsgemäße Zubereitung 4-(tert.-Butyl)-4'-methoxydibenzoylmethan, so ist es erfindungsgemäß vorteilhaft, diese Verbindung in einer Konzentration von 0,1 bis 5 Gewichts-%, bezogen auf das Gesamtgewicht der Zubereitung einzusetzen.

Enthält die erfindungsgemäße Zubereitung 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine), so ist es erfindungsgemäß vorteilhaft, diese Verbindung in einer Konzentration von 0,1 bis 4,5 Gewichts-%, bezogen auf das Gesamtgewicht der Zubereitung einzusetzen.

Erfindungsgemäß vorteilhaft enthält die erfindungsgemäße Zubereitung Natriumpolyacrylat (INCI Sodium Polyacrylate).

Erfindungsgemäß vorteilhafte Ausführungsformen der vorliegenden Erfindung sind dadurch gekennzeichnet, dass die Zubereitung Titandioxid enthält.

Erfindungsgemäß besonders bevorzugt ist ein Titandioxid in der Kristallstruktur Rutil mit einer Primärpartikelgröße von 2 -100 nm.
Es ist erfindungsgemäß ganz bevorzugt, wenn die Primärpartikelgröße des erfindungsgemäßen Titandioxides im Bereich zwischen 5 und 50 nm liegt.

Erfindungsgemäß vorteilhafte Ausführungsformen der vorliegenden Erfindung sind dabei dadurch gekennzeichnet, dass das Titandioxid mit Silica (Siliziumdioxid und/oder Kieselsäure) beschichtet ist.

Es ist dabei erfindungsgemäß bevorzugt, wenn das mit Silica beschichtete Titandioxid auf der äußeren Seite der Silica-Schicht (d.h. auf der dem Titandioxid-abgewandten Seite) eine Schicht aus Dimethicone, Simethicone oder Methicone aufweist. Unter diesen Stoffen ist Dimethicone dabei erfindungsgemäß besonders bevorzugt.

Es ist erfindungsgemäß vorteilhaft, wenn die sekundäre Partikelgröße des Titandioxid in der Kristallstruktur Rutil mit einer Primärpartikelgröße von 2 -100 nm zwischen 0,05 und 50 µm beträgt. Dabei ist der Bereich von 0,1 bis 1 µm erfindungsgemäß bevorzugt.

Die primäre und sekundäre Partikelgröße entnimmt der Fachmann folgender Literaturstelle: SCCS/1516/13 Opinion on Titanium Dioxide (nano form) Colipa No S75 der Cosmetics Europe personal care association.

Erfindungsgemäß vorteilhafte Ausführungsformen der vorliegenden Erfindung sind dadurch gekennzeichnet, dass die Zubereitung einen oder mehrere der Parfümstoffe Hexylsalicylat, Linaylacetat, 2-Isobutyl-4-hydroxy-4-methyltetrahydropyran, Adipinsäurediester, Methylheptenon, alpha-Isomethylionon, Butylphenylmethylpropioal, Coumarin, Hexylcinnamal, Limonen, Diethylsuccinat, Hydroxyisohexyl 3-Cyclohexencarboxaldehyd, Diethylsuccinat, Menthyl PCA und Citronellylmethylcrotonat, Benzyl Benzoate, Alpha-Isomethylionon, Benzylalkohol, Benzylcinnamat, Benzylsalicylat, Linalool, Citronellol, Eugenol, Geraniol enthält.

Es ist erfindungsgemäß von Vorteil, wenn die Zubereitung frei ist von 2-Ethylhexyl-2-cyano-3,3-diphenylacrylat; 4-Methoxyzimtsäure(2-ethylhexyl)ester; 4-Methoxyzimtsäureisoamylester2-Hydroxy-4-methoxybenzophenon; 2-Hydroxy-4-methoxy-4'-methylbenzophenon; 3-(4-Methylbenzyliden)campher und 3-Benzylidencampher, Propyl- und Butyl-Parabenen, Isothiazolinonen und 3-Iodpropargyl-*N*-butylcarbamat (IPBC).

Dabei führt insbesondere der Verzicht auf von 2-Ethylhexyl-2-cyano-3,3-diphenylacrylat zu einer verbesserten Auswaschbarkeit aus Textilien.

Die erfindungsgemäße Zubereitung stellt üblicherweise eine Öl-in-Wasser-Emulsion (O/W-Emulsion) dar. Diese kann die für derartige Zubereitungen üblichen Inhaltsstoffe enthalten.

Die erfindungsgemäße Zubereitung kann vorteilhaft Feuchthaltemittel enthalten. Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) und /oder Talkum und/oder Polyethylen, Nylon, Silica Diemthyl Silyate.

Es ist erfindungsgemäß bevorzugt, wenn die erfindungsgemäße Zubereitung Silica Dimethyl Silylate enthält.

Erfindungsgemäß vorteilhafte Ausführungsformen der vorliegenden Erfindung sind dadurch gekennzeichnet, dass die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

Dabei ist es erfindungsgemäß bevorzugt, wenn die Zubereitung Dibutyladipat, Dicaprylylcarbonat und/oder C12-C15 Alkylbenzoat enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol oder Polyole niedriger C-Zahl sowie deren Ether, vorzugsweise Propylenglykol, Glycerin, Elektrolyte, Selbstbräuner sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCI-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCI: Ammonium Acryloyldimethyltaurate/VP Copolymer).

Erfindungsgemäß vorteilhafte Ausführungsformen der vorliegenden Erfindung sind dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Glycyrrhitinsäure, Polidocanol, natürliche und/oder synthetische Isoflavonoide, Flavonoide, Kreatin, Kreatinin, Taurin, β-Alanin, Panthenol, Magnolol, Honokiol, Tocopherol, Tocopherylacetat, Dihydroxyaceton; 8-Hexadecen-1,16-dicarbonsäure, Glycerylglycose, (2-Hydroxyethyl)harnstoff, Hyaluronsäure und/oder deren Salze und/oder Licochalcon A enthält.

### Vergleichsversuch

Mit Hilfe des folgenden Vergleichsversuches konnte der erfinderische Effekt beispielhaft belegt werden: Es wurden die folgenden Rezepturen hergestellt und die Auswaschbarkeit aus Textilien bestimmt.

Es wurden die folgenden Rezepturen hergestellt:

| | **Octocrylene-frei** | | **Octocrylene-haltig** | |
|---|---|---|---|---|
| | **VTH50TINE 46** | **VTH50TINE 47** | **VTH50TINE 48** | **VTH50TINE 49** |
| **INCI** | **m [%]** | **m [%]** | **m [%]** | **m [%]** |
| Tocopheryl Acetate | 0,06 | 0,06 | 0,06 | 0,06 |
| Ethylhexylglycerin | 0,30 | 0,30 | | |
| Glycyrrhetinic Acid | 0,10 | 0,10 | 0,10 | 0,10 |
| Hydroxyacetophenone | | | 0,40 | 0,40 |
| Dibutyl Adipate | 2,00 | 2,00 | | |
| Hydrogenated Coco-Glycerides | | | 1,00 | 1,00 |
| Copernicia Cerifera Cera | 1,50 | 1,50 | 1,00 | 1,00 |
| Glyceryl Stearate | | | 0,25 | 0,25 |
| Polyglyceryl-10 Stearate | 0,50 | 0,50 | 0,50 | 0,50 |
| VP/Hexadecene Copolymer | 1,00 | 1,00 | 0,50 | 0,50 |
| Triacontanyl PVP | 1,00 | | 1,00 | |
| Parfum | 0,52 | 0,52 | 0,60 | 0,60 |
| Glycerin + Aqua | 1,00 | 1,00 | 0,90 | 0,90 |
| Aqua + Sodium Hydroxide | 0,10 | 0,10 | 0,20 | 0,20 |
| Phenoxyethanol | 0,50 | 0,50 | | |
| Hydroxyethylcellulose | | | 0,05 | 0,05 |
| Xanthan Gum | 0,12 | 0,12 | 0,20 | 0,20 |
| Acrylates/C10-30 Alkyl Acrylate Crosspolymer | 0,10 | 0,10 | | |
| Sodium Polyacrylate | 0,08 | 0,08 | 0,08 | 0,08 |
| Microcrystalline Cellulose + Cellulose Gum | 1,00 | 1,00 | | |
| Cellulose Gum | 0,50 | 0,50 | 0,01 | 0,01 |
| Aqua | 57,88 | 58,88 | 55,65 | 56,65 |
| Alcohol Denat. + Aqua | 5,00 | 5,00 | 5,00 | 5,00 |
| Aqua + Trisodium EDTA | 1,00 | 1,00 | 1,00 | 1,00 |
| Tetrasodium Iminodisuccinate | 0,75 | 0,75 | 0,01 | 0,01 |
| Butyl Methoxydibenzoylmethane | 4,75 | 4,75 | 4,75 | 4,75 |
| Phenylbenzimidazole Sulfonic Acid | 0,50 | 0,50 | 0,50 | 0,50 |
| Ethylhexyl Salicylate | 4,75 | 4,75 | 4,75 | 4,75 |
| Ethylhexyl Triazone | 3,00 | 3,00 | | |
| Octocrylene | | | 8,00 | 8,00 |
| Homosalate | 9,00 | 9,00 | 9,50 | 9,50 |
| Bis-Ethylhexyloxyphenol Methoxyphenyl Triazine | 3,00 | 3,00 | 4,00 | 4,00 |
| | | | | |
| **Summen:** | **100,00** | **100,00** | **100,00** | **100,00** |

### Messung der Auswaschbarkeit

Es wurden die vier Sonnenschutzemulsionen hinsichtlich der Bildung von gelben Flecken auf Baumwolltextilien über einen in vitro Auftragungs-/ Waschzyklus untersucht. Es wurden dabei weiße vorgewaschene Baumwollmonitore (100% Baumwolle) verwendet.

Dazu wurden je 3 mg/cm² der Test-Formulierung gleichmäßig auf PMMA Schönberg Platten (5,0 x 5,0 cm) verteilt und direkt mittels Andruck auf das Testtextil übertragen. Im Anschluss wurden die verfleckten Baumwollproben für 12h unter Laborbedingungen an der Luft getrocknet.

Nach der Trocknung erfolgte eine farbmetrische Charakterisierung der entstandenen Initial-Verfleckung durch Messung des Gelbgrades mit dem Farbmessgerät DATACOLOR 800 (Datacolor International).
Messgeometrie: d/8°, SCE (Glanzkomponente ausgeschlossen)
Lichtart/Beobachter: D65/10°(entsprechend mittleres Tageslicht)
UV-Filterung: an D65 angepasst, Ganz/Griesser Methode
Messöffnung: LAV (30 mm Durchmesser)
Probenhintergrund: Unterlagepapier ohne optischen Aufheller, Prüfklima: 21°C (±1°C), 41% (±4%) rel. Luftfeuchte.

Zur Auswertung wurde die Veränderung des b-Wertes aus dem CIE-Lab Farbmesssystem herangezogen. Die B-Achse charakterisiert im CIE-Lab System den Farbeindruck Gelb/ Blau, wobei positive b-Werte für eine Zunahme des Gelbanteils stehen. Je höher der b-Wert desto größer ist der Gelbeindruck.

Nach dem Messvorgang erfolgte eine separate Wäsche der Testlappen in einer Waschmaschine) (60°C, 2h, Ariel Compact Pulverwaschmittel, saubere Beiladung).

Nach Trocknung für 12h unter Laborbedingungen erfolgte erneut eine farbmetrische Charakterisierung der entstandenen Verfleckung durch Messung der Farbwerte wie bereits beschrieben mit dem Farbmessgerät DATACOLOR 800 (Datacolor International).

Die CIE-Lab System oder L*a*b*-Farbraum ist ein dreidimensionaler Messraum, in dem alle wahrnehmbaren Farben enthalten sind. Der Farbraum ist auf Grundlage der Gegenfarbentheorie konstruiert. Eine der wichtigsten Eigenschaften des L*a*b*-Farbmodells ist seine Geräteunabhängigkeit, das heißt, die Farben werden unabhängig von der Art ihrer Erzeugung und Wiedergabetechnik definiert.

Die entsprechende EU-Richtlinie ist DIN EN ISO 11664-4 "Farbmetrik - Teil 4: CIE 1976 L*a*b* Farbenraum". Die Koordinaten der CIELAB-Ebene werden gebildet aus dem Rot/ Grün-Wert a und dem Gelb/ Blau-Wert b. Die Helligkeitsachse L steht senkrecht auf dieser Ebene. Nach DIN 6174 sind L, a und b mit * zu schreiben, um sich gegen andere, z.B. das "Hunter-Lab"-System abzugrenzen.

**Ergebnis**

| | **VTH50TINE 46** | **VTH50TINE 47** | **VTH50TINE 48** | **VTH50TINE 49** |
|---|---|---|---|---|
| dB-Wert vor dem Waschen | 12,71 | 12,7 | 12,33 | 12,24 |
| dB-Wert nach dem Waschen | 5,95 | 7,83 | 7,13 | 7,65 |

**Fazit:** Die Baumwolllappen mit den Zubereitungen VTH50TINE 46 und 48 (mit Triacontanyl PVP) zeigen nach dem Waschen eine geringere Gelbfärbung als die Baumwolllappen, die mit einer Triacontanyl PVP freien Zubereitung verschmutzt waren. Wird auf Octocrylen als UV-Filter verzichtet, fällt der Effekt noch stärker aus.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | **Rezeptur 1** |
|---|---|
| **INCI** | **m [%]** |
| Ethylhexylglycerin | 0,30 |
| Glycyrrhetinic Acid | 0,10 |
| Dibutyl Adipate | 1,00 |
| Copernicia Cerifera Cera | 1,50 |
| Polyglyceryl-10 Stearate | 0,50 |
| VP/Hexadecene Copolymer | 1,00 |
| Triacontanyl PVP | 2,00 |
| Parfum | 0,52 |
| Glycerin + Aqua | 5,00 |
| Aqua + Sodium Hydroxide | 0,10 |
| Phenoxyethanol | 0,25 |
| Xanthan Gum | 0,12 |
| Acrylates/C10-30 Alkyl Acrylate Crosspolymer | 0,10 |
| Sodium Polyacrylate | 0,08 |
| Microcrystalline Cellulose + Cellulose Gum | 1,00 |
| Cellulose Gum | 0,50 |
| Aqua | q.s |
| Alcohol Denat. + Aqua | 5,00 |
| Aqua + Trisodium EDTA | 1,00 |
| Tetrasodium Iminodisuccinate | 0,75 |
| Butyl Methoxydibenzoylmethane | 4,75 |
| Phenylbenzimidazole Sulfonic Acid | 0,50 |
| Ethylhexyl Salicylate | 4,75 |
| Ethylhexyl Triazone | 3,00 |
| Homosalate | 7,00 |
| Bis-Ethylhexyloxyphenol Methoxyphenyl Triazine | 3,00 |
| **Summen:** | **100,00** |

| | **Rezeptur 2** |
|---|---|
| **INCI** | **m [%]** |
| Hydroxyacetophenone | 0,40 |
| Hydrogenated Coco-Glycerides | 0,50 |
| Copernicia Cerifera Cera | 1,50 |
| Glyceryl Stearate | 0,25 |
| Polyglyceryl-10 Stearate | 0,50 |
| VP/Hexadecene Copolymer | 0,50 |
| Triacontanyl PVP | 0,75 |
| Parfum | 0,60 |
| Glycerin + Aqua | 3,00 |
| Aqua + Sodium Hydroxide | 0,20 |
| Hydroxyethylcellulose | 0,10 |
| Xanthan Gum | 0,30 |
| Sodium Polyacrylate | 0,08 |
| Cellulose Gum | 0,01 |
| Aqua | q.s |
| Alcohol Denat. + Aqua | 5,00 |
| Aqua + Trisodium EDTA | 1,00 |
| Tetrasodium Iminodisuccinate | 0,01 |
| Butyl Methoxydibenzoylmethane | 4,75 |
| Phenylbenzimidazole Sulfonic Acid | 0,50 |
| Ethylhexyl Salicylate | 4,00 |
| Octocrylene | 8,75 |
| Homosalate | 9,50 |
| Bis-Ethylhexyloxyphenol Methoxyphenyl Triazine | 1,00 |
| **Summen:** | **100,00** |

| | **Rezeptur 3** |
|---|---|
| **INCI** | **m [%]** |
| Hydroxyacetophenone | 0,40 |
| Hydrogenated Coco-Glycerides | 1,50 |
| Copernicia Cerifera Cera | 1,50 |
| Glyceryl Stearate | 0,25 |
| Polyglyceryl-10 Stearate | 0,50 |
| VP/Hexadecene Copolymer | 0,50 |
| Triacontanyl PVP | 0,75 |
| Parfum | 0,60 |
| Glycerin + Aqua | 5,00 |
| Aqua + Sodium Hydroxide | 0,20 |
| Hydroxyethylcellulose | 0,10 |
| Xanthan Gum | 0,30 |
| Sodium Polyacrylate | 0,08 |
| Cellulose Gum | 0,01 |
| Aqua | q.s |
| Alcohol Denat. + Aqua | 5,00 |
| Aqua + Trisodium EDTA | 1,00 |
| Tetrasodium Iminodisuccinate | 0,01 |
| Butyl Methoxydibenzoylmethane | 4,75 |
| Phenylbenzimidazole Sulfonic Acid | 0,50 |
| Ethylhexyl Salicylate | 1,00 |
| Octocrylene | 8,75 |
| Homosalate | 9,50 |
| Bis-Ethylhexyloxyphenol Methoxyphenyl Triazine | 1,00 |
| **Summen:** | **100,00** |

| | **Rezeptur 4** |
|---|---|
| **INCI** | **m [%]** |
| Tocopheryl Acetate | 0,06 |
| Ethylhexylglycerin | 0,15 |
| Glycyrrhetinic Acid | 0,10 |
| Hydrogenated Coco-Glycerides | 1,00 |
| Glyceryl Stearate | 1,00 |
| Polyglyceryl-10 Stearate | 0,60 |
| Silica Dimethyl Silylate | 1,00 |
| Tapioca Starch + Aqua | 1,00 |
| VP/Hexadecene Copolymer | 0,50 |
| Parfum | 0,50 |
| Glycerin + Aqua | 0,90 |
| Phenoxyethanol | 0,30 |
| Behenyl Alcohol | 0,50 |
| Hydroxyethylcellulose | 0,30 |
| Xanthan Gum | 0,40 |
| Sodium Polyacrylate | 0,05 |
| Cellulose Gum | 0,50 |
| Aqua | q.s |
| Alcohol Denat. + Aqua | 7,00 |
| Aqua + Trisodium EDTA | 1,00 |
| Tetrasodium Iminodisuccinate | 0,01 |
| Butyl Methoxydibenzoylmethane | 3,00 |
| Ethylhexyl Salicylate | 3,00 |
| Octocrylene | 6,00 |
| Homosalate | 6,00 |
| Titanium Dioxide (nano) + Silica + Dimethicone | 1,00 |
| Triacontanyl PVP | 1,00 |
| **Summen:** | **100,00** |

| | **Rezeptur 5** |
|---|---|
| **INCI** | **m [%]** |
| Tocopheryl Acetate | 0,06 |
| Polyglyceryl-2 Caprate | 0,30 |
| Glycyrrhetinic Acid | 0,10 |
| Hydrogenated Coco-Glycerides | 1,00 |
| Glyceryl Stearate | 1,00 |
| Polyglyceryl-10 Stearate | 0,80 |
| Silica Dimethyl Silylate | 1,00 |
| VP/Hexadecene Copolymer | 0,50 |
| Parfum | 0,50 |
| Glycerin + Aqua | 0,90 |
| Phenoxyethanol | 0,30 |
| Behenyl Alcohol | 0,50 |
| Hydroxyethylcellulose | 0,20 |
| Xanthan Gum | 0,40 |
| Sodium Polyacrylate | 0,10 |
| Cellulose Gum | 0,50 |
| Aqua | q.s |
| Alcohol Denat. + Aqua | 7,00 |
| Aqua + Trisodium EDTA | 1,00 |
| Tetrasodium Iminodisuccinate | 0,01 |
| Butyl Methoxydibenzoylmethane | 4,75 |
| Octocrylene | 8,00 |
| Homosalate | 9,50 |
| Titanium Dioxide (nano) + Silica + Dimethicone | 4,00 |
| Triacontanyl PVP | 0,50 |
| **Summen:** | **100,00** |

| | **Rezeptur 6** |
|---|---|
| **INCI** | **m [%]** |
| Tocopheryl Acetate | 0,06 |
| Polyglyceryl-2 Caprate | 0,30 |
| Glycyrrhetinic Acid | 0,10 |
| Hydrogenated Coco-Glycerides | 1,00 |
| Glyceryl Stearate | 1,00 |
| Polyglyceryl-10 Stearate | 0,60 |
| Silica Dimethyl Silylate | 0,50 |
| VP/Hexadecene Copolymer | 0,50 |
| Parfum | 0,60 |
| Glycerin + Aqua | 0,90 |
| Aqua + Sodium Hydroxide | 0,20 |
| Phenoxyethanol | 0,30 |
| Behenyl Alcohol | 0,50 |
| Hydroxyethylcellulose | 0,10 |
| Xanthan Gum | 0,40 |
| Hydroxypropyl Methylcellulose | 0,20 |
| Sodium Polyacrylate | 0,08 |
| Cellulose Gum | 0,20 |
| Aqua | q.s |
| Alcohol Denat. + Aqua | 7,00 |
| Aqua + Trisodium EDTA | 1,00 |
| Tetrasodium Iminodisuccinate | 0,01 |
| Butyl Methoxydibenzoylmethane | 4,75 |
| Phenylbenzimidazole Sulfonic Acid | 0,50 |
| Ethylhexyl Salicylate | 4,75 |
| Octocrylene | 8,00 |
| Homosalate | 9,50 |
| Titanium Dioxide (nano) + Silica + Dimethicone | 4,00 |
| Triacontanyl PVP | 0,75 |
| **Summen:** | **100,00** |

## Patentansprüche

1. Verfahren zur Erhöhung der Auswaschbarkeit von Hexyl 2-[4-(diethylamino)-2-hydroxybenzoyl]benzoate (INCI: Diethylamino hydroxybenzoyl hexyl benzoate), 4-(tert.-Butyl)-4'-methoxydibenzoylmethan und/oder 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) aus Textilien, die mit einer kosmetischen Zubereitung, welche diese UV-Filter enthält, kontaminiert sind, **dadurch gekennzeichnet, dass** der kosmetischen Zubereitung 1-Ethenyl-pyrrolidin-2-on Polymer mit 1-Triaconten (INCI Triacontanyl PVP, CAS 136445-69-7) zugesetzt wird.

2. Verfahren zur Reduzierung der durch die UV-Lichtschutzfilter Hexyl 2-[4-(diethylamino)-2-hydroxybenzoyl]benzoate (INCI: Diethylamino hydroxybenzoyl hexyl benzoate), 4-(tert.-Butyl)-4'-methoxydibenzoylmethan und/oder 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) enthaltenden kosmetischen Zubereitungen hervorgerufenen Textilverfleckung, **dadurch gekennzeichnet, dass** dem Kosmetikum Zubereitung 1-Ethenyl-pyrrolidin-2-on Polymer mit 1-Triaconten (INCI Triacontanyl PVP, CAS 136445-69-7) zugesetzt wird.

3. Verwendung von 1-Ethenyl-pyrrolidin-2-on Polymer mit 1-Triaconten (INCI Triacontanyl PVP, CAS 136445-69-7) in die UV-Lichtschutzfilter Hexyl 2-[4-(diethylamino)-2-hydroxybenzoyl]benzoate (INCI: Diethylamino hydroxybenzoyl hexyl benzoate), 4-(tert.-Butyl)-4'-methoxydibenzoylmethan und/oder 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) enthaltenden kosmetischen Zubereitungen zur Erleichterung der Auswaschbarkeit der UV-Lichtschutzfilter aus mit den Zubereitungen kontaminierten Textilien.

4. Kosmetische Zubereitung enthaltend
a) ein oder mehrere UV-Filter, gewählt aus der Gruppe der Verbindungen Hexyl 2-[4-(diethylamino)-2-hydroxybenzoyl]benzoate (INCI: Diethylamino hydroxybenzoyl hexyl benzoate), 4-(tert.-Butyl)-4'-methoxydibenzoylmethan, 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine),
b) Polyglyceryl-10 Stearat (INCI Polyglyceryl-10 Stearate),
c) 1-Ethenyl-pyrrolidin-2-on Polymer mit 1-Triaconten (INCI Triacontanyl PVP, CAS 136445-69-7).

5. Kosmetische Zubereitung, Verfahren oder Verwendung nach einem der vorhergehenden Ansprüche, **dadurch gekennzeichnet, dass** die Zubereitung 1-Ethenyl-pyrrolidin-2-on Polymer mit 1-Triaconten (INCI Triacontanyl PVP, CAS 136445-69-7) in einer Konzentration von 0,01 bis 3 Gewichts-%, bezogen auf das Gesamtgewicht der Zubereitung enthält.

6. Kosmetische Zubereitung, Verfahren oder Verwendung nach einem der vorhergehenden Ansprüche, **dadurch gekennzeichnet, dass** die Zubereitung Polyglyceryl-10 Stearat in einer Konzentration von 0,01 bis 2 Gewichts-%, bezogen auf das Gesamtgewicht der Zubereitung enthält.

7. Kosmetische Zubereitung, Verfahren oder Verwendung nach einem der vorhergehenden Ansprüche, **dadurch gekennzeichnet, dass** die Zubereitung Phenoxyethanol enthält.

8. Kosmetische Zubereitung, Verfahren oder Verwendung nach einem der vorhergehenden Ansprüche, **dadurch gekennzeichnet, dass** die Zubereitung Cellulosegummi (INCI Cellulose gum) und/oder Xanthangummi (INCI Xanthan gum) enthält.

9. Kosmetische Zubereitung, Verfahren oder Verwendung nach einem der vorhergehenden Ansprüche, **dadurch gekennzeichnet, dass** die Zubereitung Tetranatriumiminodisuccinat (INCI Tetrasodium Iminodisuccinate) enthält.

10. Zubereitung, Verfahren oder Verwendung nach einem der vorhergehenden Ansprüche, **dadurch gekennzeichnet, dass** die Zubereitung Ethylhexylglycerin, Propylenglycol, Butylenglycol, 2-Methylpropan-1,3-diol, 1,2-Pentandiol, 1,2-Hexandiol, 1,2-Octandiol und/oder 1,2-Decandiol enthält.

11. Zubereitung, Verfahren oder Verwendung nach einem der vorhergehenden Ansprüche, **dadurch gekennzeichnet, dass** die Zubereitung Titandioxid enthält.

12. Zubereitung, Verwendung oder Verfahren nach einem der vorhergehenden Ansprüche, **dadurch gekennzeichnet, dass** die Zubereitung frei ist von 2-Ethylhexyl-2-cyano-3,3-diphenylacrylat; 4-Methoxyzimtsäure(2-ethylhexyl)ester; 4-Methoxyzimtsäureisoamylester2-Hydroxy-4-methoxybenzophenon; 2-Hydroxy-4-methoxy-4'-methylbenzophenon; 3-(4-Methylbenzyliden)campher und 3-Benzylidencampher, Propyl- und Butyl-Parabenen, Isothiazolinonen und 3-Iodpropargyl-*N*-butylcarbamat (IPBC).

13. Zubereitung, Verwendung oder Verfahren nach einem der vorhergehenden Ansprüche, **dadurch gekennzeichnet, dass** die Zubereitung einen oder mehrere Parfümstoffe gewählt aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Eugenol, Evernia Furfuracea Extract, Evernia Prunastri Extract, Ethylenbrassylat, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat, Vanillin enthält.

## Claims

1. Method for increasing the ability to wash out hexyl 2-[4-(diethylamino)-2-hydroxybenzoyl]benzoate (INCI: Diethylamino Hydroxybenzoyl Hexyl Benzoate), 4-(tert-butyl)-4'-methoxydibenzoylmethane and/or 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) from textiles which have been contaminated with a cosmetic preparation comprising these UV filters, **characterized in that** 1-ethenylpyrrolidin-2-one polymer with 1-triacontene (INCI: Triacontanyl PVP, CAS 136445-69-7) is added to the cosmetic preparation.

2. Method for reducing textile staining caused by cosmetic preparations comprising the UV photoprotective filters hexyl 2-[4-(diethylamino)-2-hydroxybenzoyl]benzoate (INCI: Diethylamino Hydroxybenzoyl Hexyl Benzoate), 4-(tert-butyl)-4'-methoxydibenzoylmethane and/or 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine), **characterized in that** 1-ethenylpyrrolidin-2-one polymer with 1-triacontene (INCI: Triacontanyl PVP, CAS 136445-69-7) is added to the cosmetic preparation.

3. Use of 1-ethenylpyrrolidin-2-one polymer with 1-triacontene (INCI: Triacontanyl PVP, CAS 136445-69-7) in cosmetic preparations comprising the UV photoprotective filters hexyl 2-[4-(diethylamino)-2-hydroxybenzoyl]benzoate (INCI: Diethylamino Hydroxybenzoyl Hexyl Benzoate), 4-(tert-butyl)-4'-methoxydibenzoylmethane and/or 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) for facilitating the ability of the UV photoprotective filters to be washed out from textiles contaminated with the preparations.

4. Cosmetic preparation comprising
a) one or more UV filters selected from the group of compounds comprising hexyl 2-[4-(diethylamino)-2-hydroxybenzoyl]benzoate (INCI: Diethylamino Hydroxybenzoyl Hexyl Benzoate), 4-(tert-butyl)-4'-methoxydibenzoylmethane, 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine),
b) polyglyceryl-10 stearate (INCI: Polyglyceryl-10 Stearate),
c) 1-ethenylpyrrolidin-2-one polymer with 1-triacontene (INCI: Triacontanyl PVP, CAS 136445-69-7) .

5. Cosmetic preparation, method or use according to any of the preceding claims, **characterized in that** the preparation comprises 1-ethenylpyrrolidin-2-one polymer with 1-triacontene (INCI: Triacontanyl PVP, CAS 136445-69-7) at a concentration of 0.01 to 3% by weight, based on the total weight of the preparation.

6. Cosmetic preparation, method or use according to any of the preceding claims, **characterized in that** the preparation comprises polyglyceryl-10 stearate at a concentration of 0.01 to 2% by weight, based on the total weight of the preparation.

7. Cosmetic preparation, method or use according to any of the preceding claims, **characterized in that** the preparation comprises phenoxyethanol.

8. Cosmetic preparation, method or use according to any of the preceding claims, **characterized in that** the preparation comprises cellulose gum (INCI Cellulose Gum) and/or xanthan gum (INCI Xanthan Gum).

9. Cosmetic preparation, method or use according to any of the preceding claims, **characterized in that** the preparation comprises tetrasodium iminodisuccinate (INCI Tetrasodium Iminodisuccinate).

10. Preparation, method or use according to any of the preceding claims, **characterized in that** the preparation comprises ethylhexylglycerin, propylene glycol, butylene glycol, 2-methylpropane-1,3-diol, 1,2-pentanediol, 1,2-hexanediol, 1,2-octanediol and/or 1,2-decanediol.

11. Preparation, method or use according to any of the preceding claims, **characterized in that** the preparation comprises titanium dioxide.

12. Preparation, method or use according to any of the preceding claims, **characterized in that** the preparation is free of 2-ethylhexyl 2-cyano-3,3-diphenylacrylate; 2-ethylhexyl 4-methoxycinnamate; isoamyl 4-methoxycinnamate, 2-hydroxy-4-methoxybenzophenone; 2-hydroxy-4-methoxy-4'-methylbenzophenone; 3-(4-methylbenzylidene)camphor and 3-benzylidenecamphor, propyl and butyl parabens, isothiazolinones and 3-iodopropargyl N-butylcarbamate (IPBC).

13. Preparation, method or use according to any of the preceding claims, **characterized in that** the preparation comprises one or more perfumes selected from the group of compounds comprising limonene, citral, linalool, alpha-isomethyl ionone, geraniol, citronellol, 2-isobutyl-4-hydroxy-4-methyltetrahydrofuran, 2-tert-pentylcyclohexyl acetate, 3-methyl-5-phenyl-1-pentanol, 7-acetyl-1,1,3,4,4,6-hexamethyltetralin, adipic esters, alpha-amyl cinnamaldehyde, alpha-methyl ionone, amyl C butylphenyl methylpropional cinnamal, amyl salicylate, amyl cinnamyl alcohol, anise alcohol, benzoin, benzyl alcohol, benzyl benzoate, benzyl cinnamate, benzyl salicylate, bergamot oil, bitter orange oil, butylphenyl methylpropional, cardamom oil, cedrol, cinnamal, cinnamyl alcohol, citronellyl methylcrotonate, lemon oil, coumarin, diethyl succinate, ethyl linalool, Evernia furfuracea extract, Evernia prunastri extract, ethylene brassylate, farnesol, guaiac wood oil, hexyl cinnamal, hexyl salicylate, hydroxycitronellal, lavender oil, limonene oil, lemon oil, linalyl acetate, mandarin oil, menthyl PCA, methylheptenone, nutmeg oil, rosemary oil, sweet orange oil, terpineol, tonka bean oil, triethyl citrate, vanillin.

## Revendications

1. Procédé d'augmentation de la rinçabilité de 2-[4-(diéthylamino)-2-hydroxybenzoyl]benzoate d'hexyle (INCI : Diethylamino Hydroxybenzoyl Hexyl Benzoate), de 4-(tert.-butyl)-4'-méthoxydibenzoylméthane et/ou de 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine) à partir de textiles qui sont contaminés avec une préparation cosmétique qui contient ces filtres UV, **caractérisé en ce qu'**un polymère de 1-éthényl-pyrrolidin-2-one avec du 1-triacontène (INCI : Triacontanyl PVP, CAS 136445-69-7) est ajouté à la préparation cosmétique.

2. Procédé visant à réduire les taches sur des textiles causées par des préparations cosmétiques contenant les filtres de protection contre la lumière UV 2-[4-(diéthylamino)-2-hydroxybenzoyl]benzoate d'hexyle (INCI : Diethylamino Hydroxybenzoyl Hexyl Benzoate), 4-(tert.-butyl)-4'-méthoxydibenzoylméthane et/ou 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine), **caractérisé en ce qu'**un polymère de 1-éthényl-pyrrolidin-2-one avec du 1-triacontène (INCI : Triacontanyl PVP, CAS 136445-69-7) est ajouté à la préparation cosmétique.

3. Utilisation d'un polymère de 1-éthényl-pyrrolidin-2-one avec du 1-triacontène (INCI : Triacontanyl PVP, CAS 136445-69-7) dans des préparations cosmétiques contenant les filtres de protection contre la lumière UV 2-[4-(diéthylamino)-2-hydroxybenzoyl]benzoate d'hexyle (INCI : Diethylamino Hydroxybenzoyl Hexyl Benzoate), 4-(tert.-butyl)-4'-méthoxydibenzpylméthane et/ou 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine) pour faciliter la rinçabilité des filtres de protection contre la lumière UV à partir de textiles contaminés avec les préparations.

4. Préparation cosmétique contenant :
a) un ou plusieurs filtres UV, choisis dans le groupe de composés constitué par le 2-[4-(diéthylamino)-2-hydroxybenzoyl]benzoate d'hexyle (INCI : Diethylamino Hydroxybenzoyl Hexyl Benzoate), le 4-(tert.-butyl)-4'-méthoxydibenzoylméthane et/ou le 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazine),
b) du stéarate de polyglycéryle-10 (INCI : Polyglycéryl-10 Stearate)
c) un polymère de 1-éthényl-pyrrolidin-2-one avec du 1-triacontène (INCI : Triacontanyl PVP, CAS 136445-69-7) .

5. Préparation cosmétique, procédé ou utilisation selon l'une quelconque des revendications précédentes, **caractérisé en ce que** la préparation contient le polymère de 1-éthényl-pyrrolidin-2-one avec du 1-triacontène (INCI : Triacontanyl PVP, CAS 136445-69-7) en une concentration de 0,01 à 3 % en poids, par rapport au poids total de la préparation.

6. Préparation cosmétique, procédé ou utilisation selon l'une quelconque des revendications précédentes, **caractérisé en ce que** la préparation contient le stéarate de polyglycéryle-10 en une concentration de 0,01 à 2 % en poids, par rapport au poids total de la préparation.

7. Préparation cosmétique, procédé ou utilisation selon l'une quelconque des revendications précédentes, **caractérisé en ce que** la préparation contient du phénoxyéthanol.

8. Préparation cosmétique, procédé ou utilisation selon l'une quelconque des revendications précédentes, **caractérisé en ce que** la préparation contient de la gomme de cellulose (INCI : Cellulose gum) et/ou de la gomme xanthane (INCI : Xanthan gum).

9. Préparation cosmétique, procédé ou utilisation selon l'une quelconque des revendications précédentes, **caractérisé en ce que** la préparation contient de l'iminodisuccinate de tétrasodium (INCI : Tetrasodium Iminodisuccinate).

10. Préparation, procédé ou utilisation selon l'une quelconque des revendications précédentes, **caractérisé en ce que** la préparation contient de l'éthylhexylglycérine, du propylène glycol, du butylène glycol, du 2-méthylpropane-1,3-diol, du 1,2-pentanediol, du 1,2-hexanediol, du 1,2-octanediol et/ou du 1,2-décanediol.

11. Préparation, procédé ou utilisation selon l'une quelconque des revendications précédentes, **caractérisé en ce que** la préparation contient du dioxyde de titane.

12. Préparation, utilisation ou procédé selon l'une quelconque des revendications précédentes, **caractérisé en ce que** la préparation est exempte d'acrylate de 2-éthylhexyl-2-cyano-3,3-diphényle, d'ester 2-éthylhexylique de l'acide 4-méthoxycinnamique, d'ester isoamylique de l'acide 4-méthoxycinnamique, de 2-hydroxy-4-méthoxybenzophénone, de 2-hydroxy-4-méthoxy-4'-méthylbenzophénone, de 3-(4-méthylbenzylidène)-camphre et de 3-benzylidène-camphre, de propyl- et butyl-parabènes, d'isothiazolinones et de carbamate de 3-iodopropargyl-N-butyle (IPBC).

13. Préparation, utilisation ou procédé selon l'une quelconque des revendications précédentes, **caractérisé en ce que** la préparation contient un ou plusieurs parfums choisis dans le groupe de composés constitué par le limonène, le citral, le linalool, l'alpha-isométhylionone, le géraniol, le citronellol, le 2-isobutyl-4-hydroxy-4-méthyltétrahydropyrane, l'acétate de 2-tert-pentylcyclohexyle, le 3-méthyl-5-phényl-1-pentanol, la 7-acétyl-1,1,3,'4,4,6-hexaméthyltétraline, le diester de l'acide adipique, l'alpha-amylcinnamaldéhyde, l'alpha-méthylionone, l'amyl C butylphénylméthylpropionalcinnamal, le salicylate d'amyle, l'alcool amylcinnamylique, l'alcool anisique, la benzoïne, l'alcool benzylique, le benzoate de benzyle, le cinnamate de benzyle, le salicylate de benzyle, l'huile de bergamote, l'huile d'orange amère, le butylphénylméthylpropioal, l'huile de cardamome, le cédrol, le cinnamal, l'alcool cinnamylique, le crotonate de citronellylméthyle, l'huile de citron, la coumarine, le succinate de diéthyle, l'éthyllinalool, l'eugénol, l'extrait d'Evernia Furfuracea, l'extrait d'Evernia Prunastri, le brassylate d'éthylène, le farnésol, l'huile de bois de gaïac, l'hexylcinnamal, le salicylate d'hexyle, l'hydroxycitronellal, l'huile de lavande, l'huile de citron, l'acétate de linalyle, l'huile de mandarine, le menthyl PCA, la méthylhepténone, l'huile de noix de muscade, l'huile de romarin, l'huile d'orange douce, le terpinéol, l'huile de fève de Tonka, le citrate de triéthyle, la vanilline.
